# EUROPEAN PATENT APPLICATION

(11) **EP 4 273 166 A1**
(43) Date of publication of application: **08.11.2023**
(21) Application number: 21914461.5
(22) Date of filing: 29.12.2021
(51) Int. Cl.: C07K 16/28, A61K 39/00, A61P 35/00

(54) **ANTI-FGFR2 ANTIBODY AND USE THEREOF**

(30) Priority: 29.12.2020 CN 202011600351
(71) Applicant: Shenzhen Forward Pharmaceuticals Co., Limited, Shenzhen, Guangdong 518067 (CN)
(72) Inventor: ZHU, Chenggang, Shenzhen, Guangdong 518067 (CN); XU, Liangliang, Shenzhen, Guangdong 518067 (CN); DONG, Jiexian, Shenzhen, Guangdong 518067 (CN); ZHANG, Chaochun, Shenzhen, Guangdong 518067 (CN); YANG, Xuan, Shenzhen, Guangdong 518067 (CN); PENG, Fangli, Shenzhen, Guangdong 518067 (CN); LI, Yeqing, Shenzhen, Guangdong 518067 (CN); CHEN, Chaole, Shenzhen, Guangdong 518067 (CN); BAO, Liming, Shenzhen, Guangdong 518067 (CN); MIN, Xiangyan, Shenzhen, Guangdong 518067 (CN)
(74) Representative: Pharma Patents International AG
(86) International application number: PCT/CN2021/142321
(87) International publication number: WO 2022/143728

(57) **Abstract**

An anti-FGFR2 antibody, an encoding nucleic acid of the antibody, a vector and host cell for the expression and production thereof, an antibody-drug conjugate, and a pharmaceutical composition comprising the antibody. The present invention further relates to the use of the antibody molecule in a drug for diagnosing a cancer caused by FGFR2-pathway-related dysregulation, and the use of the antibody or antibody-drug conjugate in the preparation of a medicament for treating a cancer caused by FGFR2-pathway-related dysregulation, in particular gastric cancer.

## Description

### Technical Field

The invention relates to antibody molecules, specifically human anti-FGFR2 antibody molecules, especially anti-FGFR2111b antibody molecules.

### Background Art

Fibroblast growth factor receptor 2 (FGFR2) is a tyrosine kinase receptor, having an extracellular region with two immunoglobulin-like domains (β-isomer) (D2 and D3 domains, respectively) or three immunoglobulin-like domains (α-isomer) (D1, D2 and D3 domains, respectively), which is linked to a transmembrane region and an intracellular dityrosine kinase subdomain. Depending on the source of exons, FGFR2 can be divided into IIIb and IIIc subtypes which are different mainly at the D3 domain. The IIIb subtype is divided into FGFR2αIIIb (having three immunoglobulin-like domains D 1, D2 and D3) and FGFR2βIIIb (having two immunoglobulin-like domains D2 and D3) based on the number of immunoglobulin-like structures in the extracellular region. In addition, the IIIb subtype is mainly expressed in epithelial tissues, and the IIIc subtype is mainly expressed in mesenchymal tissues. Some FGF ligands of the two receptors have opposite expression patterns, for example, FGF7, FGF10 and FGF22 that bind to FGFR2IIIb are expressed in mesenchymal tissues, while FGF4, FGF5 and FGF6 that bind to FGFR2IIIc are expressed in epithelial cells; therefore, it is supposed that FGFR2 plays an important role in epithelial-mesenchymal transition.

Based on sequence homology, FGFR1, FGFR3 and FGFR4 are also members of the same family as FGFR2. The activation of the signaling pathway of this family requires fibroblast growth factors (FGFs) as ligands, in which FGF binds to the receptor FGFR2 mainly through the D2 and D3 domains of the receptor, while also binds to heparan sulfate proteoglycans, inducing FGFR dimerization and autophosphorylation, thereby transducing RAS-ERK and PI3K-AKT signaling cascades through FGFR substrate 2 (FRS2) and calmodulin signaling pathway PLCA, and also involving DAG-PKC and IP3 signaling cascades. Abnormal activation of FGFR signals is associated with various malignant tumors.

In normal cells, FGFR2 is located on chromosome 10q26 and mainly participates in cell differentiation, proliferation, and apoptosis during tissue repair and development. Studies have shown that knocking out the FGFR2IIIb gene in mice can lead to embryonic lethality. Furthermore, a large amount of evidences show that overexpression of FGFR2 or FGFs and changes in genes, such as gene amplification, gene fusion and rearrangement, gene point mutation and chromosomal translocation, will lead to dysregulated FGFR2 signaling pathway, and the dysregulated FGFR/FGF signaling pathway is closely related to cell carcinogenesis. Potential overexpression of FGFR2, activation of missense mutation, or abnormal protein fusion have been reported in a variety of cancer types, including endometrial cancer, ovarian cancer, breast cancer, lung cancer, gastric cancer, esophageal cancer, bladder cancer, and cholangiocarcinoma. For example, FGFR2IIIb is highly expressed in 40% of gastric cancer tissue samples, and FGFR2IIIb gene amplification has a mutation frequency of up to 15% in the gastric cancer patient population. Compared to patients without FGFR2IIIb gene amplification, gastric cancer patients with overexpression of FGFR2IIIb due to FGFR2IIIb gene amplification have significant lymph node metastasis, which is significantly associated with poorly differentiated gastric adenocarcinoma and a lower survival rate, making the gene amplification a very poor prognostic indicator for gastric cancer patients. FGFR2 gene fusion and rearrangement were found in about 9%-14% of cholangiocarcinoma patients.

Taken together, FGFR2 can become a potential target for tumor treatment, blockers such as antibodies can be used to block the binding of FGF and FGFR2 in the treatment, thus inhibiting the function of FGFR/FGF signaling pathway, and this treatment idea has been proved effective in other tyrosine kinase (such as HER2 and EGFR) positive tumors.

### Summary of the Invention

The invention provides a set of novel antibodies against FGFR2IIIb. The invention also provides a pharmaceutical composition comprising the anti-FGFR2IIIb antibody described in the invention, and the use of the antibody in the manufacture of a medicament for diagnosing or treating gastric cancer, in particular gastric cancer where the FGFR2IIIb gene amplification leads to overexpression of FGFR2IIIb.

Specifically, the invention provides an anti-FGFR2IIIb antibody. The preferred embodiment of the invention is an antibody or antigen-binding fragment thereof that specifically binds to FGFR2IIIb, comprising heavy chain CDR1, CDR2 and CDR3 and light chain CDR1, CDR2 and CDR3 which have at least 80%, preferably at least 90%, more preferably at least 95% identity respectively with the heavy chain CDR1, CDR2 and CDR3 and the light chain CDR1, CDR2 and CDR3 sequences of an antibody selected from the group of FWB 1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB1924 and FWB1925. More preferably, the invention relates to an antibody or antigen-binding fragment thereof that specifically binds to FGFR2IIIb, comprising heavy chain CDR1, CDR2 and CDR3 and light chain CDR1, CDR2 and CDR3 of an antibody selected from the group of: FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB1924 and FWB1925.

A more preferred embodiment of the invention is an antibody or antigen-binding fragment thereof that specifically binds to FGFR2IIIb, comprising a heavy chain variable region and a light chain variable region, wherein the heavy chain variable region and the light chain variable region have at least 85%, preferably at least 90%, more preferably 95%, and most preferably 98% identity with heavy chain variable region and light chain variable region sequences of an antibody selected from the group of: FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB1924 and FWB1925. Further more preferably, the invention relates to an antibody or antigen-binding fragment thereof that specifically binds to FGFR2IIIb, comprising heavy chain variable region and light chain variable region sequences of an antibody selected from the group of: FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB1924 and FWB1925.

An especially preferred embodiment of the invention is an antibody comprising three heavy chain CDRs and three light chain CDRs of an antibody molecule referred to in this article as FWB1913, FWB1914, and FWB1925.

The invention also provides an isolated nucleic acid encoding the antibody or antigen-binding fragment thereof described in the invention.

The invention also provides an expression vector comprising the isolated nucleic acid described in the invention. The invention also provides a host cell comprising the nucleic acid or the expression vector described in the invention, wherein the host cell is preferably a eukaryotic host cell, and more preferably a mammalian host cell.

The invention also provides a composition comprising a first nucleic acid and a second nucleic acid, wherein the first nucleic acid encodes a heavy chain of the antibody described in the invention, and the second nucleic acid encodes a light chain of the antibody described in the invention.

The invention provides a pharmaceutical composition for treating an FGFR2IIIb-related disease or disorder, comprising the antibody or antigen-binding fragment thereof, the isolated nucleic acid, the expression vector, or the host cell described in the invention, and further comprising a pharmaceutical carrier. Preferably, the FGFR2IIIb-related disease or disorder is gastric cancer, in particular gastric cancer where the FGFR2IIIb gene amplification leads to overexpression of FGFR2IIIb.

The invention also relates to an antibody-drug conjugate (ADC). The invention also relates to a pharmaceutical composition comprising the antibody-drug conjugate and a pharmaceutical carrier. The antibody-drug conjugate comprises the antibody or antigen-binding fragment thereof described in the invention and a drug.

The invention also relates to the use of the antibody or antigen-binding fragment thereof or the antibody-drug conjugate described in the invention in the manufacture of a medicament for treating a cancer caused by FGFR2-pathway-related dysregulation, preferably gastric cancer, and most preferably gastric cancer where the FGFR2IIIb gene amplification leads to overexpression of FGFR2IIIb.

### Detailed Description of Embodiments

As used in the description and the appended claims, the singular forms "a", "an" and "the" include plural referents unless the context clearly specifies otherwise. Thus, for example, reference to "a molecule" optionally includes a combination of two or more such molecules, and the like.

As used herein, the term "about" refers to the usual error range for the respective numerical value readily known to the skilled person in this technical field. Reference to "about" a numerical value or parameter herein includes (and describes) embodiments that are directed to that numerical value or parameter per se.

The terms used herein have the commonly known meaning in the art, unless otherwise stated.

The FGFR2IIIb referred to herein usually refers to human FGFR2IIIb, which is also referred to as "antigen" in several parts herein, unless otherwise specified. The invention provides an antibody against human FGFR2IIIb. The anti-FGFR2IIIb antibody can be an antibody that specifically binds to FGFR2IIIb, in particular mammalian (such as human) FGFR2IIIb. The antibody molecule can be an isolated antibody molecule.

In any embodiment described in this application, the antibody can bind to FGFR2IIIb rather than FGFR2IIIc.

In one embodiment, the anti-FGFR2IIIb antibody molecule comprises a heavy chain and a light chain. The antigen-binding fragment of the anti-FGFR2IIIb antibody is an Fab fragment, an F(ab') fragment, an Fv fragment, an F(ab')2 fragment, a single chain antibody (scFV), and a diabody.

The anti-FGFR2IIIb antibody molecule of the invention can be an effective-inhuman, human, humanized, CDR-grafted, chimeric, mutated, affinity-matured, deimmunized, synthetic, or *in vitro* produced antibody molecule. In one embodiment, the anti-FGFR2IIIb antibody is a humanized antibody. In another embodiment, the antibody molecule has a heavy chain constant region selected from the heavy chain constant region of such as IgG1, IgG2, IgG3, IgG4, IgM, IgA1, IgA2, IgD and IgE, particularly the heavy chain constant region of IgG1, IgG2, IgG3 and IgG4, and more particularly the heavy chain constant region of IgG1 (such as human IgG1). The heavy chain constant region is generally of human or a modified form of human constant region. In another embodiment, the antibody molecule has a light chain constant region selected from such as Lambda or Kappa (preferably Lambda, such as human Lambda) light chain constant region. In one embodiment, the constant region can be altered, e.g., mutated, to modify the properties of the antibody molecule (e.g., to increase or decrease one or more of: Fc receptor binding, antibody glycosylation, the number of cysteine residues, effector cell function, or complement function).

The invention provides the use of the anti-FGFR2IIIb antibody described in the invention in the manufacture of a medicament for treating gastric cancer. In some embodiments, the gastric cancer comprises FGFR2 gene amplification. In some embodiments, the FGFR2 amplification comprises a FGFR2 : CEN10 (centromere of chromosome 10) ratio of > 3. In some embodiments, the cancer overexpresses FGFR2. In some embodiments, for a cancer comprising FGFR2 amplification comprising a FGFR2IIIb : CEN10 (centromere of chromosome 10) ratio of > 3, the degree of overexpression of FGFR2IIIb is higher than that of FGFR2IIIc. In some embodiments, for a cancer comprising FGFR2 amplification, the normalized level of FGFR2IIIb expression exceeds that of FGFR2IIIc expression by 2, 3, 5 or 10 times. In some embodiments, the expression level is normalized to GUSB. In some embodiments, the cancer overexpresses FGFR2IIIb but does not comprise FGFR2 gene amplification. In some embodiments, the expression or overexpression of FGFR2IIIb is determined by IHC. In some embodiments, 1+, 2+ or 3+ staining of tumor cells by IHC indicates the overexpression of FGFR2IIIb. In some embodiments, 2+ or 3+ staining in tumor cells by IHC indicates the overexpression of FGFR2IIIb.

An antibody-drug conjugate (ADC) is a small molecule drug with biological activity that is linked to a monoclonal antibody via a chemical linkage, in which the monoclonal antibody serves as a vehicle to target and transport the small molecule drug to a target cell. The antibody-drug conjugate of the invention is made by chemically linking the anti-FGFR2IIIb antibody of the invention to a drug.

The FGF/FGFR signaling pathway is related to cell proliferation, differentiation, apoptosis, and migration. Activating mutations in FGFR or overexpression of ligands/receptors in tumor cells result in persistent activation of the signaling pathway, which is not only closely related to the occurrence, proliferation, poor prognosis and the like of various malignant tumors, but also plays an important role in tumor neovascularization, tumor invasion and metastasis and other processes. The anti-FGFR2IIIb antibody of the invention can inhibit the abnormal activation of the FGFR/FGF signaling pathway in tumor cells by blocking the binding of FGFR2IIIb to its ligand FGF (which encompasses FGF1, FGF7 (KGF), and other members of the FGF7 subfamily such as FGF3, FGF10 and FGF22), thereby inhibiting tumor cell proliferation, as well as the neogenesis, differentiation, and migration of tumor vascular endothelial cells.

Tables 1 and 2 list the sequences of six CDRs of the antibodies described in the invention.

**Table 1. The sequences of the heavy chain CDRs of the antibodies of the invention**

| **Antibody No. FWB** | **HCDR1** | **HCDR1 SEQ ID NO:** | **HCDR2** | **HCDR2 SEQ ID NO:** | **HCDR3** | **HCDR3 SEQ ID NO:** |
|---|---|---|---|---|---|---|
| FWB1904 | SYNVH | 1 | SIYPDNGDTSYNQNFRG | 2 | GDFAY | 3 |
| FWB1905 | SYNVH | 7 | SIYPDNGDSSYNQNYKG | 8 | GDFAY | 9 |
| FWB1906 | SYNVH | 13 | SIYPDNGDSSYNQNYRG | 14 | GDFAY | 15 |
| FWB1907 | SYNVN | 19 | SIYPDNGDSSYNNNYKG | 20 | GDFAY | 21 |
| FWB1908 | TYNVH | 25 | SIYPDNGDSTYNQNFKG | 26 | GDFAY | 27 |
| FWB1910 | TYNVH | 31 | SIYPDNGDTSYDEDFKG | 32 | GDFAY | 33 |
| FWB1911 | SYNVH | 37 | SIYPDNGDSSYNQNYKG | 38 | GDFAY | 39 |
| FWB1912 | SYNVH | 43 | SIYPDNGDSSYNQNYKG | 44 | G DYAY | 45 |
| FWB1913 | SYNVH | 49 | SIYPDNGDSSYNQNYKG | 50 | G DYAY | 51 |
| FWB1914 | SYNVH | 55 | SIYPDNGDSSYNNNYKG | 56 | GDFAY | 57 |
| FWB1915 | SYNVH | 61 | SIYPDNGDSSYDEDYKG | 62 | GDFAY | 63 |
| FWB1916 | SYNVH | 67 | SIYPDNGDSSYNQNYKG | 68 | GDFAY | 69 |
| FWB1918 | SYNVH | 73 | SIYPDNGDSSYNQNFRG | 74 | GDFAY | 75 |
| FWB1919 | SYNVH | 79 | SIYPDNGDSSYNQNYRG | 80 | GDFAY | 81 |
| FWB1920 | SYNIH | 85 | SIYPDNGDSSYNQNYRG | 86 | GDFAY | 87 |
| FWB1921 | SYNVH | 91 | SIYPDNGDSTYNQNYRG | 92 | GDFAY | 93 |
| FWB1922 | SYNVH | 97 | SIYPDNGDSTYNQNYRG | 98 | GDFAY | 99 |
| FWB1923 | SYNVH | 103 | SIYPDNGDSTYDEDFKG | 104 | GDFAY | 105 |
| FWB1924 | SYNVH | 109 | SLYPDNGDTSYDEDYKG | 110 | GDFAY | 111 |
| FWB1925 | SYNVH | 115 | SIYPDNGDSTYDEDYRG | 116 | GDFAY | 117 |

**Table 2. The sequences of the light chain CDRs of the antibodies of the invention**

| Antibody No. FWB | **LCDR1** | **LCDR1 SEQ ID NO:** | **LCDR2** | **LCDR2 SEQ ID NO:** | **LCDR3** | **LCDR3 SEQ ID NO:** |
|---|---|---|---|---|---|---|
| FWB1904 | KASNGISNDIA | 4 | SASYRYS | 5 | QQHSTTPYT | 6 |
| FWB1905 | KASNGVSNDIA | 10 | SASYRYS | 11 | QQHSTTPYT | 12 |
| FWB1906 | KASNGISNDIA | 16 | SASYRYS | 17 | QQHSTTPYT | 18 |
| FWB1907 | RASNGISNDIA | 22 | SASYRYS | 23 | QQHSTTPYT | 24 |
| FWB1908 | KGSQGVSNDVA | 28 | SASYRYT | 29 | QQHSTTPYT | 30 |
| FWB1910 | KVSQGVSNDAV | 34 | SASYRYT | 35 | QQHSTTPYT | 36 |
| FWB1911 | KASNGVSNDIA | 40 | SASYRYS | 41 | QQHSTTPYS | 42 |
| FWB1912 | KASNGVSNDIA | 46 | SASYRYS | 47 | QQHSTTPYT | 48 |
| FWB1913 | KASNGVSNDIA | 52 | SASYRYS | 53 | QQHSTTPYS | 54 |
| FWB1914 | KASNGVSNDIA | 58 | SASYRYS | 59 | QQHSTTPYT | 60 |
| FWB1915 | KASNGVSNDIA | 64 | SASYRYS | 65 | QQHSTTPYT | 66 |
| FWB1916 | KASNGISNDIA | 70 | SASYRYS | 71 | QQHSTTPYT | 72 |
| FWB1918 | KGSNGISNDIA | 76 | SASYRYS | 77 | QQHSTTPYT | 78 |
| FWB1919 | RGSNGISNDIA | 82 | SASYRYS | 83 | QQHSTTPYT | 84 |
| FWB1920 | KGSNGVSNDIA | 88 | SASYRYS | 89 | QQHSTTPYT | 90 |
| FWB1921 | KGSNGVSNDIA | 94 | SASYRYS | 95 | QQHSTTPYT | 96 |
| FWB1922 | KGSNGISNDIA | 100 | SASYRYS | 101 | QQHSTTPYT | 102 |
| FWB1923 | KVSQGVSNDAV | 106 | SASYRYS | 107 | QQHSTTPYT | 108 |
| FWB1924 | KVSQGVSNDAV | 112 | SASYRYS | 113 | QQHSTTPYT | 114 |
| FWB1925 | KGSNGISNDIA | 118 | SASYRYS | 119 | QQHSTTPYT | 120 |

**Table 3. The sequences of the heavy chain variable regions and light chain variable regions of the antibodies of the invention**

| Antibody No. FWB | VH sequence | VH No. SEQ ID NO: | VL sequence | VL No. SEQ ID NO: |
|---|---|---|---|---|
| 1904 | QVQLVQSGAEVKKPGSSVKVSCKASGYI FTSYNVHWVRQAPGQGLEWIGSIYPDN GDTSYNQNFRGRATITADKSTSTAYMEL SSLRSEDTAVYYCARGDFAYWGQGTLVT VSS | 121 | DIQMTQSPSSLSASVGDRVTITCKASNGISND IAWYQQKPGKAPKLLIYSASYRYSGVPSRFSG SGSGTDFTFTISSLQPEDIATYYCQQHSTTPYT FGQGTKLEIK | 122 |
| 1905 | QVQLVQSGAEVKKPGSSVKVSCKASGYI FTSYNVHWVRQAPGQGLEWIGSIYPDN GDSSYNQNYKGRATITADKSTSTAYMEL SSLRSEDTAVYYCARGDFAYWGQGTLVT VSS | 123 | DIQMTQSPSSLSASVGDRVTITCKASNGVSN DIAWYQQKPGKAPKLLIYSASYRYSGVPSRFS GSGSGTDFTFTISSLQPEDIATYYCQQHSTTP YTFGQGTKLEIK | 124 |
| 1906 | QVQLVQSGAEVKKPGSSVKVSCKASGYI FTSYNVHWVRQAPGQGLEWIGSIYPDN GDSSYNQNYRGRATITADKSTSTAYMEL SSLRSEDTAVYYCARGDFAYWGQGTLVT VSS | 125 | DIQMTQSPSSLSASVGDRVTITCKASNGISND IAWYQQKPGKAPKLLIYSASYRYSGVPSRFSG SGSGTDFTFTISSLQPEDIATYYCQQHSTTPYT FGQGTKLEIK | 126 |
| 1907 | QVQLVQSGAEVKKPGSSVKVSCKASGYI FTSYNVNWVRQAPGQGLEWIGSIYPDN GDSSYNNNYKGRATITADKSTSTAYMEL SSLRSEDTAVYYCARGDFAYWGQGTLVT VSS | 127 | DIQMTQSPSSLSASVGDRVTITCRASNGISND IAWYQQKPGKAPKLLIYSASYRYSGVPSRFSG SGSGTDFTFTISSLQPEDIATYYCQQHSTTPYT FGQGTKLEIK | 128 |
| 1908 | QVQLVQSGAEVKKPGSSVKVSCKASGYI FTTYNVHWVRQAPGQGLEWIGSIYPDN | 129 | DIQMTQSPSSLSASVGDRVTITCKGSQGVSN DVAWYQQKPGKAPKLLIYSASYRYTGVPSRF | 130 |
| | GDSTYNQNFKGRATITADKSTSTAYMEL SSLRSEDTAVYYCARGDFAYWGQGTLVT VSS | | SGSGSGTDFTFTISSLQPEDIATYYCQQHSTTP YTFGQGTKLEIK | |
| 1910 | QVQLVQSGAEVKKPGSSVKVSCKASGYI FTTYNVHWVRQAPGQGLEWIGSIYPDN GDTSYDEDFKGRATITADKSTSTAYMELS SLRSEDTAVYYCARGDFAYWGQGTLVT VSS | 131 | DIQMTQSPSSLSASVGDRVTITCKVSQGVSN DAVWYQQKPGKAPKLLIYSASYRYTGVPSRF SGSGSGTDFTFTISSLQPEDIATYYCQQHSTTP YTFGQGTKLEIK | 132 |
| 1911 | QVQLVQSGAEVKKPGSSVKVSCKASGYI FTSYNVHWVRQAPGQGLEWIGSIYPDN GDSSYNQNYKGRATITADKSTSTAYMEL SSLRSEDTAVYYCARGDFAYWGQGTLVT VSS | 133 | DIQMTQSPSSLSASVGDRVTITCKASNGVSN DIAWYQQKPGKAPKLLIYSASYRYSGVPSRFS GSGSGTDFTFTISSLQPEDIATYYCQQHSTTP YSFGQGTKLEIK | 134 |
| 1912 | QVQLVQSGAEVKKPGSSVKVSCKASGYI FTSYNVHWVRQAPGQGLEWIGSIYPDN GDSSYNQNYKGRATITADKSTSTAYMEL SSLRSEDTAVYYCARGDYAYWGQGTLVT VSS | 135 | DIQMTQSPSSLSASVGDRVTITCKASNGVSN DIAWYQQKPGKAPKLLIYSASYRYSGVPSRFS GSGSGTDFTFTISSLQPEDIATYYCQQHSTTP YTFGQGTKLEIK | 136 |
| 1913 | QVQLVQSGAEVKKPGSSVKVSCKASGYI FTSYNVHWVRQAPGQGLEWIGSIYPDN GDSSYNQNYKGRATITADKSTSTAYMEL SSLRSEDTAVYYCARGDYAYWGQGTLVT VSS | 137 | DIQMTQSPSSLSASVGDRVTITCKASNGVSN DIAWYQQKPGKAPKLLIYSASYRYSGVPSRFS GSGSGTDFTFTISSLQPEDIATYYCQQHSTTP YSFGQGTKLEIK | 138 |
| 1914 | QVQLVQSGAEVKKPGSSVKVSCKASGYI FTSYNVHWVRQAPGQGLEWIGSIYPDN GDSSYNNNYKGRATITADKSTSTAYMEL SSLRSEDTAVYYCARGDFAYWGQGTLVT VSS | 139 | DIQMTQSPSSLSASVGDRVTITCKASNGVSN DIAWYQQKPGKAPKLLIYSASYRYSGVPSRFS GSGSGTDFTFTISSLQPEDIATYYCQQHSTTP YTFGQGTKLEIK | 140 |
| 1915 | QVQLVQSGAEVKKPGSSVKVSCKASGYI FTSYNVHWVRQAPGQGLEWIGSIYPDN GDSSYDEDYKGRATITADKSTSTAYMELS SLRSEDTAVYYCARGDFAYWGQGTLVT VSS | 141 | DIQMTQSPSSLSASVGDRVTITCKASNGVSN DIAWYQQKPGKAPKLLIYSASYRYSGVPSRFS GSGSGTDFTFTISSLQPEDIATYYCQQHSTTP YTFGQGTKLEIK | 142 |
| 1916 | QVQLVQSGAEVKKPGSSVKVSCKASGYI FTSYNVHWVRQAPGQGLEWIGSIYPDN GDSSYNQNYKGRATITADKSTSTAYMEL SSLRSEDTAVYYCARGDFAYWGQGTLVT | 143 | DIQMTQSPSSLSASVGDRVTITCKASNGISND IAWYQQKPGKAPKLLIYSASYRYSGVPSRFSG SGSGTDFTFTISSLQPEDIATYYCQQHSTTPYT FGQGTKLEIK | 144 |
| | VSS | | | |
| 1918 | QVQLVQSGAEVKKPGSSVKVSCKASGYI FTSYNVHWVRQAPGQGLEWIGSIYPDN GDSSYNQNFRGRATITADKSTSTAYMEL SSLRSEDTAVYYCARGDFAYWGQGTLVT VSS | 145 | DIQMTQSPSSLSASVGDRVTITCKGSNGISND IAWYQQKPGKAPKLLIYSASYRYSGVPSRFSG SGSGTDFTFTISSLQPEDIATYYCQQHSTTPYT FGQGTKLEIK | 146 |
| 1919 | QVQLVQSGAEVKKPGSSVKVSCKASGYI FTSYNVHWVRQAPGQGLEWIGSIYPDN GDSSYNQNYRGRATITADKSTSTAYMEL SSLRSEDTAVYYCARGDFAYWGQGTLVT VSS | 147 | DIQMTQSPSSLSASVGDRVTITCRGSNGISN DIAWYQQKPGKAPKLLIYSASYRYSGVPSRFS GSGSGTDFTFTISSLQPEDIATYYCQQHSTTP YTFGQGTKLEIK | 148 |
| 1920 | QVQLVQSGAEVKKPGSSVKVSCKASGYI FTSYNIHWVRQAPGQGLEWIGSIYPDN GDSSYNQNYRGRATITADKSTSTAYMEL SSLRSEDTAVYYCARGDFAYWGQGTLVT VSS | 149 | DIQMTQSPSSLSASVGDRVTITCKGSNGVSN DIAWYQQKPGKAPKLLIYSASYRYSGVPSRFS GSGSGTDFTFTISSLQPEDIATYYCQQHSTTP YTFGQGTKLEIK | 150 |
| 1921 | QVQLVQSGAEVKKPGSSVKVSCKASGYI FTSYNVHWVRQAPGQGLEWIGSIYPDN GDSTYNQNYRGRATITADKSTSTAYMEL SSLRSEDTAVYYCARGDFAYWGQGTLVT VSS | 151 | DIQMTQSPSSLSASVGDRVTITCKGSNGVSN DIAWYQQKPGKAPKLLIYSASYRYSGVPSRFS GSGSGTDFTFTISSLQPEDIATYYCQQHSTTP YTFGQGTKLEIK | 152 |
| 1922 | QVQLVQSGAEVKKPGSSVKVSCKASGYI FTSYNVHWVRQAPGQGLEWIGSIYPDN GDSTYNQNYRGRATITADKSTSTAYMEL SSLRSEDTAVYYCARGDFAYWGQGTLVT VSS | 153 | DIQMTQSPSSLSASVGDRVTITCKGSNGISND IAWYQQKPGKAPKLLIYSASYRYSGVPSRFSG SGSGTDFTFTISSLQPEDIATYYCQQHSTTPYT FGQGTKLEIK | 154 |
| 1923 | QVQLVQSGAEVKKPGSSVKVSCKASGYI FTSYNVHWVRQAPGQGLEWIGSIYPDN GDSTYDEDFKGRATITADKSTSTAYMELS SLRSEDTAVYYCARGDFAYWGQGTLVT VSS | 155 | DIQMTQSPSSLSASVGDRVTITCKVSQGVSN DAVWYQQKPGKAPKLLIYSASYRYSGVPSRF SGSGSGTDFTFTISSLQPEDIATYYCQQHSTTP YTFGQGTKLEIK | 156 |
| 1924 | QVQLVQSGAEVKKPGSSVKVSCKASGYI FTSYNVHWVRQAPGQGLEWIGSLYPDN GDTSYDEDYKGRATITADKSTSTAYMELS SLRSEDTAVYYCARGDFAYWGQGTLVT VSS | 157 | DIQMTQSPSSLSASVGDRVTITCKVSQGVSN DAVWYQQKPGKAPKLLIYSASYRYSGVPSRF SGSGSGTDFTFTISSLQPEDIATYYCQQHSTTP YTFGQGTKLEIK | 158 |
| 1925 | QVQLVQSGAEVKKPGSSVKVSCKASGYI | 159 | DIQMTQSPSSLSASVGDRVTITCKGSNGISND | 160 |
| | FTSYNVHWVRQAPGQGLEWIGSIYPDN GDSTYDEDYRGRATITADKSTSTAYMELS SLRSEDTAVYYCARGDFAYWGQGTLVT VSS | | IAWYQQKPGKAPKLLIYSASYRYSGVPSRFSG SGSGTDFTFTISSLQPEDIATYYCQQHSTTPYT FGQGTKLEIK | |

**Table 4. The sequences of the heavy and light chains of the antibodies of the invention**

| Antibody No. FWB | HC | VH No. SEQ ID NO: | VC | VL No. SEQ ID NO: |
|---|---|---|---|---|
| 1904 | QVQLVQSGAEVKKPGSSVKVSCKASGYIFTSYN VHWVRQAPGQGLEWIGSIYPDNGDTSYNQNFRG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 161 | DIQMTQSPSSLSASVG DRVTITCKASNGISNDI AWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 162 |
| 1905 | QVQLVQSGAEVKKPGSSVKVSCKASGYIFTSYN VHWVRQAPGQGLEWIGSIYPDNGDSSYNQNYKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 163 | DIQMTQSPSSLSASVG DRVTITCKASNGVSND IAWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 164 |
| 1906 | QVQLVQSGAEVKKPGSSVKVSCKASGYIFTSYN VHWVRQAPGQGLEWIGSIYPDNGDSSYNQNYRG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF | 165 | DIQMTQSPSSLSASVG DRVTITCKASNGISNDI AWYQQKPGKAPKLLI YSASYRYSGVPSRFSG SGSGTDFTFTISSLQPE | 166 |
| | PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | | DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | |
| 1907 | QVQLVQSGAEVKKPGSSVKVSCKASGYIFTSYN VNWVRQAPGQGLEWIGSIYPDNGDSSYNNNYKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 167 | DIQMTQSPSSLSASVG DRVTITCRASNGISNDI AWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 168 |
| 1908 | QVQLVQSGAEVKKPGSSVKVSCKASGYIFTTYN VHWVRQAPGQGLEWIGSIYPDNGDSTYNQNFKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 169 | DIQMTQSPSSLSASVG DRVTITCKGSQGVSND VAWYQQKPGKAPKLL IYSASYRYTGVPSRFS GSGSGTDFTFTISSLQP EDIATYYCQQHSTTPY TFGQGTKLEIKRTVAA PSVFIFPPSDEQLKSGT ASVVCLLNNFYPREA KVQWKVDNALQSGNS QESVTEQDSKDSTYSL SSTLTLSKADYEKHKV YACEVTHQGLSSPVTK SFNRGEC | 170 |
| 1910 | QVQLVQSGAEVKKPGSSVKVSCKASGYIFTTYN VHWVRQAPGQGLEWIGSIYPDNGDTSYDEDFKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK | 171 | DIQMTQSPSSLSASVG DRVTITCKVSQGVSND AVWYQQKPGKAPKLL IYSASYRYTGVPSRFS GSGSGTDFTFTISSLQP EDIATYYCQQHSTTPY | 172 |
| | PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | | TFGQGTKLEIKRTVAA PSVFIFPPSDEQLKSGT ASVVCLLNNFYPREA KVQWKVDNALQSGNS QESVTEQDSKDSTYSL SSTLTLSKADYEKHKV YACEVTHQGLSSPVTK SFNRGEC | |
| 1911 | QVQLVQSGAEVKKPGSSVKVSCKASGYIFTSYN VHWVRQAPGQGLEWIGSIYPDNGDSSYNQNYKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 173 | DIQMTQSPSSLSASVG DRVTITCKASNGVSND IAWYQQKPGKAPKLLI YSASYRYSGVPSRFSG SGSGTDFTFTISSLQPE DIATYYCQQHSTTPYS FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 174 |
| 1912 | QVQLVQSGAEVKKPGSSVKVSCKASGYIFTSYN VHWVRQAPGQGLEWIGSIYPDNGDSSYNQNYKG RATITADKSTSTAYMELSSLRSEDTAVYYCARGD YAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 175 | DIQMTQSPSSLSASVG DRVTITCKASNGVSND IAWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 176 |
| 1913 | QVQLVQSGAEVKKPGSSVKVSCKASGYIFTSYN VHWVRQAPGQGLEWIGSIYPDNGDSSYNQNYKG RATITADKSTSTAYMELSSLRSEDTAVYYCARGD YAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV | 177 | DIQMTQSPSSLSASVG DRVTITCKASNGVSND IAWYQQKPGKAPKLLI YSASYRYSGVPSRFSG SGSGTDFTFTISSLQPE DIATYYCQQHSTTPYS FGQGTKLEIKRTVAAP SVFIFPPSDEQLKSGTA | 178 |
| | KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | | SVVCLLNNFYPREAK VQWKVDNALQSGNSQ ESVTEQDSKDSTYSLS STLTLSKADYEKHKV YACEVTHQGLSSPVTK SFNRGEC | |
| 1914 | QVQLVQSGAEVKKPGSSVKVSCKASGYIFTSYN VHWVRQAPGQGLEWIGSIYPDNGDSSYNNNYKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 179 | DIQMTQSPSSLSASVG DRVTITCKASNGVSND IAWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 180 |
| 1915 | QVQLVQSGAEVKKPGSSVKVSCKASGYIFTSYN VHWVRQAPGQGLEWIGSIYPDNGDSSYDEDYKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 181 | DIQMTQSPSSLSASVG DRVTITCKASNGVSND IAWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 182 |
| 1916 | QVQLVQSGAEVKKPGSSVKVSCKASGYIFTSYN VHWVRQAPGQGLEWIGSIYPDNGDSSYNQNYKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA | 183 | DIQMTQSPSSLSASVG DRVTITCKASNGISNDI AWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ | 184 |
| | KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | | ESVTEQDSKDSTYSLS STLTLSKADYEKHKV YACEVTHQGLSSPVTK SFNRGEC | |
| 1918 | QVQLVQSGAEVKKPGSSVKVSCKASGYIFTSYN VHWVRQAPGQGLEWIGSIYPDNGDSSYNQNFRG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 185 | DIQMTQSPSSLSASVG DRVTITCKGSNGISNDI AWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 186 |
| 1919 | QVQLVQSGAEVKKPGSSVKVSCKASGYIFTSYN VHWVRQAPGQGLEWIGSIYPDNGDSSYNQNYRG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 187 | DIQMTQSPSSLSASVG DRVTITCRGSNGISNDI AWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 188 |
| 1920 | QVQLVQSGAEVKKPGSSVKVSCKASGYIFTSYNI HWVRQAPGQGLEWIGSIYPDNGDSSYNQNYRGR ATITADKSTSTAYMELSSLRSEDTAVYYCARGDF AYWGQGTLVTVSSASTKGPSVFPLAPSSKSTSGG TAALGCLVKDYFPEPVTVSWNSGALTSGVHTFP AVLQSSGLYSLSSVVTVPSSSLGTQTYICNVNHKP SNTKVDKRVEPKSCDKTHTCPPCPAPELLGGPSV FLFPPKPKDTLMISRTPEVTCVVVDVSHEDPEVKF NWYVDGVEVHNAKTKPREEQYNSTYRVVSVLT VLHQDWLNGKEYKCKVSNKALPAPIEKTISKAK GQPREPQVYTLPPSREEMTKNQVSLTCLVKGFYP SDIAVEWESNGQPENNYKTTPPVLDSDGSFFLYS | 189 | DIQMTQSPSSLSASVG DRVTITCKGSNGVSND IAWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV | 190 |
| | KLTVDKSRWQQGNVFSCSVMHEALHNHYTQKS LSLSPGK | | YACEVTHQGLSSPVTK SFNRGEC | |
| 1921 | QVQLVQSGAEVKKPGSSVKVSCKASGYIFTSYN VHWVRQAPGQGLEWIGSIYPDNGDSTYNQNYRG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 191 | DIQMTQSPSSLSASVG DRVTITCKGSNGVSND IAWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 192 |
| 1922 | QVQLVQSGAEVKKPGSSVKVSCKASGYIFTSYN VHWVRQAPGQGLEWIGSIYPDNGDSTYNQNYRG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 193 | DIQMTQSPSSLSASVG DRVTITCKGSNGISNDI AWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 194 |
| 1923 | QVQLVQSGAEVKKPGSSVKVSCKASGYIFTSYN VHWVRQAPGQGLEWIGSIYPDNGDSTYDEDFKG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 195 | DIQMTQSPSSLSASVG DRVTITCKVSQGVSND AVWYQQKPGKAPKLL IYSASYRYSGVPSRFS GSGSGTDFTFTISSLQP EDIATYYCQQHSTTPY TFGQGTKLEIKRTVAA PSVFIFPPSDEQLKSGT ASVVCLLNNFYPREA KVQWKVDNALQSGNS QESVTEQDSKDSTYSL SSTLTLSKADYEKHKV YACEVTHQGLSSPVTK SFNRGEC | 196 |
| 1924 | QVQLVQSGAEVKKPGSSVKVSCKASGYIFTSYN VHWVRQAPGQGLEWIGSLYPDNGDTSYDEDYK GRATITADKSTSTAYMELSSLRSEDTAVYYCARG DFAYWGQGTLVTVSSASTKGPSVFPLAPSSKSTS GGTAALGCLVKDYFPEPVTVSWNSGALTSGVHT FPAVLQSSGLYSLSSVVTVPSSSLGTQTYICNVNH KPSNTKVDKRVEPKSCDKTHTCPPCPAPELLGGP SVFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 197 | DIQMTQSPSSLSASVG DRVTITCKVSQGVSND AVWYQQKPGKAPKLL IYSASYRYSGVPSRFS GSGSGTDFTFTISSLQP EDIATYYCQQHSTTPY TFGQGTKLEIKRTVAA PSVFIFPPSDEQLKSGT ASVVCLLNNFYPREA KVQWKVDNALQSGNS QESVTEQDSKDSTYSL SSTLTLSKADYEKHKV YACEVTHQGLSSPVTK SFNRGEC | 198 |
| 1925 | QVQLVQSGAEVKKPGSSVKVSCKASGYIFTSYN VHWVRQAPGQGLEWIGSIYPDNGDSTYDEDYRG RATITADKSTSTAYMELSSLRSEDTAVYYCARGD FAYWGQGTLVTVSSASTKGPSVFPLAPSSKSTSG GTAALGCLVKDYFPEPVTVSWNSGALTSGVHTF PAVLQSSGLYSLSSVVTVPSSSLGTQTYICNVNHK PSNTKVDKRVEPKSCDKTHTCPPCPAPELLGGPS VFLFPPKPKDTLMISRTPEVTCVVVDVSHEDPEV KFNWYVDGVEVHNAKTKPREEQYNSTYRVVSV LTVLHQDWLNGKEYKCKVSNKALPAPIEKTISKA KGQPREPQVYTLPPSREEMTKNQVSLTCLVKGFY PSDIAVEWESNGQPENNYKTTPPVLDSDGSFFLY SKLTVDKSRWQQGNVFSCSVMHEALHNHYTQK SLSLSPGK | 199 | DIQMTQSPSSLSASVG DRVTITCKGSNGISNDI AWYQQKPGKAPKLLI YSASYRYSGVPSRFSG SGSGTDFTFTISSLQPE DIATYYCQQHSTTPYT FGQGTKLEIKRTVAAP SVFIFPPSDEQLKSGTA SVVCLLNNFYPREAK VQWKVDNALQSGNSQ ESVTEQDSKDSTYSLS STLTLSKADYEKHKV YACEVTHQGLSSPVTK SFNRGEC | 200 |

### Examples

### Example 1: Preparation of antibodies using a genetic engineering method

Gene sequences were synthesized based on the heavy chain and light chain amino acid sequences shown in Tables 5 and 6 below, respectively, and cloned into the expression vector pcDNA3.4 (Invitrogen). The dual plasmids were co transfected into HEK293 cells through electroporation, so that the transformed HEK293 cells expressed antibodies. After culturing under shaking at 37°C for one week, the supernatant was collected to purify the antibodies. Purification was performed using a Protein A affinity chromatography column, and the purity of the antibodies was detected using SDS-PAGE and SEC-HPLC detection methods after purification, respectively. All the antibodies achieved a purity of 95% or higher. 25 candidate antibodies were screened by ELISA to obtain candidate antibodies with high binding activity to FGFR2IIIb.

**Table 5. The sequences of the heavy chain CDRs and FRs of the antibodies of Example 1**

| FWB No. | HFR1 | HCD R1 | HFR2 | HCDR2 | HFR3 | HCD R3 | HFR4 |
|---|---|---|---|---|---|---|---|
| FWB1 901 | QVQLVQSGAEVKK PGSSVKVSCKASGY IFT | TYDV H | WVRQAPGQGLE WIG | SIYPNDGDTSYN QNFKG | RATITADKSTSTAYMEL SSLRSEDTAVYYCAR | GDFA Y | WGQ GTLVT VSS |
| FWB1 902 | QVQLVQSGAEVKK PGSSVKVSCKASGY IFT | TYDV H | WVRQAPGQGLE WIG | SIYPNDGDTSYN QNFKG | RATITADKSTSTAYMEL SSLRSEDTAVYYCAR | GNFA Y | WGQ GTLVT VSS |
| FWB1 903 | QVQLVQSGAEVKK PGSSVKVSCKASGY IFT | TYDV H | WVRQAPGQGLE WIG | SIYPNNGDTSYN QNFKG | RATITADKSTSTAYMEL SSLRSEDTAVYYCAR | GNFA Y | WGQ GTLVT VSS |
| FWB1 904 | QVQLVQSGAEVKK PGSSVKVSCKASGY IFT | SYNV H | WVRQAPGQGLE WIG | SIYPDNGDTSYN QNFRG | RATITADKSTSTAYMEL SSLRSEDTAVYYCAR | GDFA Y | WGQ GTLVT VSS |
| FWB1 905 | QVQLVQSGAEVKK PGSSVKVSCKASGY IFT | SYNV H | WVRQAPGQGLE WIG | SIYPDNGDSSYN QNYKG | RATITADKSTSTAYMEL SSLRSEDTAVYYCAR | GDFA Y | WGQ GTLVT VSS |
| FWB1 906 | QVQLVQSGAEVKK PGSSVKVSCKASGY IFT | SYNV H | WVRQAPGQGLE WIG | SIYPDNGDSSYN QNYRG | RATITADKSTSTAYMEL SSLRSEDTAVYYCAR | GDFA Y | WGQ GTLVT VSS |
| FWB1 907 | QVQLVQSGAEVKK PGSSVKVSCKASGY IFT | SYNV N | WVRQAPGQGLE WIG | SIYPDNGDSSYN NNYKG | RATITADKSTSTAYMEL SSLRSEDTAVYYCAR | GDFA Y | WGQ GTLVT VSS |
| FWB1 908 | QVQLVQSGAEVKK PGSSVKVSCKASGY IFT | TYNV H | WVRQAPGQGLE WIG | SIYPDNGDSTYN QNFKG | RATITADKSTSTAYMEL SSLRSEDTAVYYCAR | GDFA Y | WGQ GTLVT VSS |
| FWB1 909 | QVQLVQSGAEVKK PGSSVKVSCKASGY IFT | TYNV H | WVRQAPGQGLE WIG | SIYPNDGDTSYN QNFKG | RATITADKSTSTAYMEL SSLRSEDTAVYYCAR | GDFA Y | WGQ GTLVT VSS |
| FWB1 910 | QVQLVQSGAEVKK PGSSVKVSCKASGY IFT | TYNV H | WVRQAPGQGLE WIG | SIYPDNGDTSYD EDFKG | RATITADKSTSTAYMEL SSLRSEDTAVYYCAR | GDFA Y | WGQ GTLVT VSS |
| FWB1 911 | QVQLVQSGAEVKK PGSSVKVSCKASGY IFT | SYNV H | WVRQAPGQGLE WIG | SIYPDNGDSSYN QNYKG | RATITADKSTSTAYMEL SSLRSEDTAVYYCAR | GDFA Y | WGQ GTLVT VSS |
| FWB1 912 | QVQLVQSGAEVKK PGSSVKVSCKASGY IFT | SYNV H | WVRQAPGQGLE WIG | SIYPDNGDSSYN QNYKG | RATITADKSTSTAYMEL SSLRSEDTAVYYCAR | GDYA Y | WGQ GTLVT VSS |
| FWB1 913 | QVQLVQSGAEVKK PGSSVKVSCKASGY IFT | SYNV H | WVRQAPGQGLE WIG | SIYPDNGDSSYN QNYKG | RATITADKSTSTAYMEL SSLRSEDTAVYYCAR | GDYA Y | WGQ GTLVT VSS |
| FWB1 914 | QVQLVQSGAEVKK PGSSVKVSCKASGY IFT | SYNV H | WVRQAPGQGLE WIG | SIYPDNGDSSYN NNYKG | RATITADKSTSTAYMEL SSLRSEDTAVYYCAR | GDFA Y | WGQ GTLVT VSS |
| FWB1 915 | QVQLVQSGAEVKK PGSSVKVSCKASGY IFT | SYNV H | WVRQAPGQGLE WIG | SIYPDNGDSSYD EDYKG | RATITADKSTSTAYMEL SSLRSEDTAVYYCAR | GDFA Y | WGQ GTLVT VSS |
| FWB1 916 | QVQLVQSGAEVKK PGSSVKVSCKASGY IFT | SYNV H | WVRQAPGQGLE WIG | SIYPDNGDSSYN QNYKG | RATITADKSTSTAYMEL SSLRSEDTAVYYCAR | GDFA Y | WGQ GTLVT VSS |
| FWB1 917 | QVQLVQSGAEVKK PGSSVKVSCKASGY IFT | SYNV H | WVRQAPGQGLE WIG | SIYPDNGDSSYN QNYRG | RATITADKSTSTAYMEL SSLRSEDTAVYYCAR | GDFA Y | WGQ GTLVT VSS |
| FWB1 918 | QVQLVQSGAEVKK PGSSVKVSCKASGY IFT | SYNV H | WVRQAPGQGLE WIG | SIYPDNGDSSYN QNFRG | RATITADKSTSTAYMEL SSLRSEDTAVYYCAR | GDFA Y | WGQ GTLVT VSS |
| FWB1 919 | QVQLVQSGAEVKK PGSSVKVSCKASGY IFT | SYNV H | WVRQAPGQGLE WIG | SIYPDNGDSSYN QNYRG | RATITADKSTSTAYMEL SSLRSEDTAVYYCAR | GDFA Y | WGQ GTLVT VSS |
| FWB1 920 | QVQLVQSGAEVKK PGSSVKVSCKASGY IFT | SYNIH | WVRQAPGQGLE WIG | SIYPDNGDSSYN QNYRG | RATITADKSTSTAYMEL SSLRSEDTAVYYCAR | GDFA Y | WGQ GTLVT VSS |
| FWB1 921 | QVQLVQSGAEVKK PGSSVKVSCKASGY IFT | SYNV H | WVRQAPGQGLE WIG | SIYPDNGDSTYN QNYRG | RATITADKSTSTAYMEL SSLRSEDTAVYYCAR | GDFA Y | WGQ GTLVT VSS |
| FWB1 922 | QVQLVQSGAEVKK PGSSVKVSCKASGY IFT | SYNV H | WVRQAPGQGLE WIG | SIYPDNGDSTYN QNYRG | RATITADKSTSTAYMEL SSLRSEDTAVYYCAR | GDFA Y | WGQ GTLVT VSS |
| FWB1 923 | QVQLVQSGAEVKK PGSSVKVSCKASGY IFT | SYNV H | WVRQAPGQGLE WIG | SIYPDNGDSTYD EDFKG | RATITADKSTSTAYMEL SSLRSEDTAVYYCAR | GDFA Y | WGQ GTLVT VSS |
| FWB1 924 | QVQLVQSGAEVKK PGSSVKVSCKASGY IFT | SYNV H | WVRQAPGQGLE WIG | SLYPDNGDTSYD EDYKG | RATITADKSTSTAYMEL SSLRSEDTAVYYCAR | GDFA Y | WGQ GTLVT VSS |
| FWB1 925 | QVQLVQSGAEVKK PGSSVKVSCKASGY IFT | SYNV H | WVRQAPGQGLE WIG | SIYPDNGDSTYD EDYRG | RATITADKSTSTAYMEL SSLRSEDTAVYYCAR | GDFA Y | WGQ GTLVT VSS |

The heavy chain constant region of all the antibodies was:

ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHT

CPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNHY
TQKSLSLSPGK

**Table 6. The sequences of the light chain CDRs and FRs of the antibodies of Example 1**

| FWB No. | LFR1 | CDR1 | LFR2 | CDR2 | LFR3 | CDR3 | LFR4 |
|---|---|---|---|---|---|---|---|
| FWB19 01 | DIQMTQSPSSLSASVGD RVTITC | KASQGVSD DVA | WYQQKPGKAP KLLIY | SASYR YV | WYQQKPGKAP KLLIY | QQHSTT PYT | FGQGTKL EIK |
| FWB19 02 | DIQMTQSPSSLSASVGD RVTITC | KASQGVSD DVA | WYQQKPGKAP KLLIY | SASYR YV | WYQQKPGKAP KLLIY | QQHSTT PYV | FGQGTKL EIK |
| FWB19 03 | DIQMTQSPSSLSASVGD RVTITC | KASQGLSND VA | WYQQKPGKAP KLLIY | SASYR YV | WYQQKPGKAP KLLIY | QQHSTT PYV | FGQGTKL EIK |
| FWB19 04 | DIQMTQSPSSLSASVGD RVTITC | KASNGISND IA | WYQQKPGKAP KLLIY | SASYR YS | WYQQKPGKAP KLLIY | QQHSTT PYT | FGQGTKL EIK |
| FWB19 05 | DIQMTQSPSSLSASVGD RVTITC | KASNGVSN DIA | WYQQKPGKAP KLLIY | SASYR YS | WYQQKPGKAP KLLIY | QQHSTT PYT | FGQGTKL EIK |
| FWB19 06 | DIQMTQSPSSLSASVGD RVTITC | KASNGISND IA | WYQQKPGKAP KLLIY | SASYR YS | WYQQKPGKAP KLLIY | QQHSTT PYT | FGQGTKL EIK |
| FWB19 07 | DIQMTQSPSSLSASVGD RVTITC | RASNGISND IA | WYQQKPGKAP KLLIY | SASYR YS | WYQQKPGKAP KLLIY | QQHSTT PYT | FGQGTKL EIK |
| FWB19 08 | DIQMTQSPSSLSASVGD RVTITC | KGSQGVSN DVA | WYQQKPGKAP KLLIY | SASYR YT | WYQQKPGKAP KLLIY | QQHSTT PYT | FGQGTKL EIK |
| FWB19 09 | DIQMTQSPSSLSASVGD RVTITC | KATQGVSN DAV | WYQQKPGKAP KLLIY | SASYR YT | WYQQKPGKAP KLLIY | QQHSTT PYT | FGQGTKL EIK |
| FWB19 10 | DIQMTQSPSSLSASVGD RVTITC | KVSQGVSN DAV | WYQQKPGKAP KLLIY | SASYR YT | WYQQKPGKAP KLLIY | QQHSTT PYT | FGQGTKL EIK |
| FWB19 11 | DIQMTQSPSSLSASVGD RVTITC | KASNGVSN DIA | WYQQKPGKAP KLLIY | SASYR YS | WYQQKPGKAP KLLIY | QQHSTT PYS | FGQGTKL EIK |
| FWB19 12 | DIQMTQSPSSLSASVGD RVTITC | KASNGVSN DIA | WYQQKPGKAP KLLIY | SASYR YS | WYQQKPGKAP KLLIY | QQHSTT PYT | FGQGTKL EIK |
| FWB19 13 | DIQMTQSPSSLSASVGD RVTITC | KASNGVSN DIA | WYQQKPGKAP KLLIY | SASYR YS | WYQQKPGKAP KLLIY | QQHSTT PYS | FGQGTKL EIK |
| FWB19 14 | DIQMTQSPSSLSASVGD RVTITC | KASNGVSN DIA | WYQQKPGKAP KLLIY | SASYR YS | WYQQKPGKAP KLLIY | QQHSTT PYT | FGQGTKL EIK |
| FWB19 15 | DIQMTQSPSSLSASVGD RVTITC | KASNGVSN DIA | WYQQKPGKAP KLLIY | SASYR YS | WYQQKPGKAP KLLIY | QQHSTT PYT | FGQGTKL EIK |
| FWB19 16 | DIQMTQSPSSLSASVGD RVTITC | KASNGISND IA | WYQQKPGKAP KLLIY | SASYR YS | WYQQKPGKAP KLLIY | QQHSTT PYT | FGQGTKL EIK |
| FWB19 | DIQMTQSPSSLSASVGD | KGSNGISND | WYQQKPGKAP | SASYR | WYQQKPGKAP | QQHSTT | FGQGTKL |
| 17 | RVTITC | IA | KLLIY | YS | KLLIY | PYT | EIK |
| FWB19 18 | DIQMTQSPSSLSASVGD RVTITC | KGSNGISND IA | WYQQKPGKAP KLLIY | SASYR YS | WYQQKPGKAP KLLIY | QQHSTT PYT | FGQGTKL EIK |
| FWB19 19 | DIQMTQSPSSLSASVGD RVTITC | RGSNGISND IA | WYQQKPGKAP KLLIY | SASYR YS | WYQQKPGKAP KLLIY | QQHSTT PYT | FGQGTKL EIK |
| FWB19 20 | DIQMTQSPSSLSASVGD RVTITC | KGSNGVSN DIA | WYQQKPGKAP KLLIY | SASYR YS | WYQQKPGKAP KLLIY | QQHSTT PYT | FGQGTKL EIK |
| FWB19 21 | DIQMTQSPSSLSASVGD RVTITC | KGSNGVSN DIA | WYQQKPGKAP KLLIY | SASYR YS | WYQQKPGKAP KLLIY | QQHSTT PYT | FGQGTKL EIK |
| FWB19 22 | DIQMTQSPSSLSASVGD RVTITC | KGSNGISND IA | WYQQKPGKAP KLLIY | SASYR YS | WYQQKPGKAP KLLIY | QQHSTT PYT | FGQGTKL EIK |
| FWB19 23 | DIQMTQSPSSLSASVGD RVTITC | KVSQGVSN DAV | WYQQKPGKAP KLLIY | SASYR YS | WYQQKPGKAP KLLIY | QQHSTT PYT | FGQGTKL EIK |
| FWB19 24 | DIQMTQSPSSLSASVGD RVTITC | KVSQGVSN DAV | WYQQKPGKAP KLLIY | SASYR YS | WYQQKPGKAP KLLIY | QQHSTT PYT | FGQGTKL EIK |
| FWB19 25 | DIQMTQSPSSLSASVGD RVTITC | KGSNGISND IA | WYQQKPGKAP KLLIY | SASYR YS | WYQQKPGKAP KLLIY | QQHSTT PYT | FGQGTKL EIK |

The light chain constant region of all the antibodies was:

### Example 2: Determination of binding activity of anti-FGFR2 antibodies by ELISA method

First, a 96-well ELISA plate was coated with 100 µL of rhFGFR2IIIb-Fc (recombinant human FGFR2IIIb-Fc) at 2 µg/mL overnight at 4°C, incubated with 250 µL of a blocking solution (3% BSA in PBST) at 37°C for 2 hours, and then washed 3 times with PBST. A 10-fold dilution series of the test antibody was made from 10 µg/mL, for a total of 8 concentration points (comprising a blank control), and the antibody diluted in the series was added to the ELISA wells at 100 µL per well and incubated at 37°C for 1 hour, followed by washing the plate 3 times with PBST. The anti-Fab HRP conjugate was added as a secondary antibody and incubated at 37°C for 1 hour. Finally, the plate was washed 3 times with PBST and 100 µL TMB was added and reacted for 15 minutes. The chromogenic reaction was stopped with 1N hydrochloric acid (50 µL), and the absorbance value at 450 nm was detected on a microplate reader (M5) to calculate the binding activity (EC₅₀) of each antibody to human FGFR2IIIb. The results were shown in Table 7, and finally FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922 and FWB1925, which have strong binding activity to human FGFR2IIIb, human FGFR2βIIIb and murine FGFR2IIIb but without binding to human FGFR2IIIc, FGFR3IIIb, FGFR3IIIc, FGFR4, FGFR1IIIb and FGFR1IIIc, were screened for use in the subsequent experiments.

**Table 7: The binding of antibodies to multiple FGFRs**

| Antibody No. | ELISA EC₅₀ (nM) | | | | | | | | |
|---|---|---|---|---|---|---|---|---|---|
| | Human FGFR2 IIIb | Human FGFR2β IIIb | Murine FGFR2I IIb | Human FGFR2I IIc | Human FGFR3I IIb | Human FGFR3I IIc | Human FGFR4 | Human FGFR1I IIb | Human FGFR1I IIc |
| FWB1904 | 0.3515 | 0.1625 | 0.3084 | -- | -- | -- | -- | -- | -- |
| FWB1905 | 0.2984 | 0.1732 | 0.2617 | -- | -- | -- | -- | -- | -- |
| FWB1906 | 0.3236 | 0.1674 | 0.2891 | -- | -- | -- | -- | -- | -- |
| FWB1907 | 1.059 | 0.2618 | 5.898 | -- | -- | -- | -- | -- | -- |
| FWB1908 | 0.2813 | 0.1258 | 0.2254 | -- | -- | -- | -- | -- | -- |
| FWB1910 | 0.2791 | 0.2099 | 0.2955 | -- | -- | -- | -- | -- | -- |
| FWB1911 | 0.6968 | 0.2464 | 0.3383 | -- | -- | -- | -- | -- | -- |
| FWB1912 | 0.7241 | 0.2069 | 0.2928 | -- | -- | -- | -- | -- | -- |
| FWB1913 | 0.9029 | 0.1962 | 0.2961 | -- | -- | -- | -- | -- | -- |
| FWB1914 | 1.006 | 0.2026 | 0.2497 | -- | -- | -- | -- | -- | -- |
| FWB1915 | 0.8769 | 0.185 | 0.2727 | -- | -- | -- | -- | -- | -- |
| FWB1916 | 0.5823 | 0.1429 | 0.2904 | -- | -- | -- | -- | -- | -- |
| FWB1918 | 0.6259 | 0.1353 | 0.2698 | -- | -- | -- | -- | -- | -- |
| FWB1919 | 0.8791 | 0.1491 | 0.3135 | -- | -- | -- | -- | -- | -- |
| FWB1920 | 1.209 | 0.1527 | 0.274 | -- | -- | -- | -- | -- | -- |
| FWB1921 | 0.6205 | 0.1762 | 0.2605 | -- | -- | -- | -- | -- | -- |
| FWB1922 | 0.6564 | 0.2153 | 0.139 | -- | -- | -- | -- | -- | -- |
| FWB1923 | 1.684 | Not detected | Not detected | Not detected | Not detected | Not detected | Not detected | Not detected | Not detected |
| FWB1924 | 1.885 | Not detected | Not detected | Not detected | Not detected | Not detected | Not detected | Not detected | Not detected |
| FWB1925 | 0.6434 | 0.1733 | 0.0908 | -- | -- | -- | -- | -- | -- |

| | | | | | | | | | |
|---|---|---|---|---|---|---|---|---|---|
| "--" refers to no binding signal | | | | | | | | | |

### Example 3: Determination of binding activity of anti-FGFR2 antibodies by FACS

The test antibodies were FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922 and FWB1925. 2×105 cells (KATO III and SNU16, purchased from ATCC, with FGFR2IIIb receptors highly expressed on the cell surface) per well were collected in the microplate, centrifuged, resuspended with PBS containing 2% FBS. The antibodies were diluted in a 5-fold gradient with 30 µg/mL as the initial concentration, and 100 µL of the antibodies with corresponding concentrations or a blank control were added to the cells in each well, and incubated at 4°C for 1 hour. After the cells were washed twice with PBS containing 2% FBS, the Alexa 488 Goat Anti-Human IgG was added, and incubated at 4°C in dark for 1 hour. The cells were washed twice with PBS containing 2% FBS, and then resuspended with 100 µL PBS containing 2% FBS, and finally the fluorescence signal on the cell surface was detected using a flow cytometer. Finally, FWB1904, FWB1905, FWB1912, FWB1914, FWB1915, FWB1916, FWB1919, FWB1921 and FWB1925 having strong binding activity to KATO III and SNU16 were screened for use in the subsequent experiments.

**Table 8: The binding ability of the antibodies to FGFR2IIIb receptor on KATO III and SNU16 cells**

| Antibody No. | ELISA EC₅₀ (nM) | |
|---|---|---|
| | KATOIII | SNU16 |
| FWB 1904 | 1.621 | 1.133 |
| FWB 1905 | 1.092 | 1.331 |
| FWB 1906 | 1.849 | 2.278 |
| FWB1908 | 2.172 | 1.604 |
| FWB1910 | 4.156 | 2.301 |
| FWB1911 | 1.958 | 3.012 |
| FWB1912 | 1.901 | 2.747 |
| FWB1913 | 1.846 | 2.169 |
| FWB1914 | 2.555 | 2.851 |
| FWB1915 | 2.188 | 2.445 |
| FWB1916 | 1.974 | 3.024 |
| FWB1918 | 1.604 | 2.959 |
| FWB1919 | 1.283 | 2.842 |
| FWB1920 | 1.83 | 4.328 |
| FWB1921 | 1.774 | 3.857 |
| FWB 1922 | 2.047 | 4.418 |
| FWB1925 | 1.958 | 4.193 |

### Example 4: Determination of the activity of the antibodies in blocking the binding of cell surface receptor FGFR2IIIb to its ligand FGF7 by FACS

The test antibodies were FWB1904, FWB1905, FWB1912, FWB1914, FWB1915, FWB1916, FWB1919, FWB1921 and FWB1925. 3×10⁵ cells (KATO III and SNU16) per well were collected in the microplate, centrifuged, resuspended with PBS containing 2% FBS. The antibodies were diluted in a 5-fold gradient with 30 µg/mL as the initial concentration, 100 µL of the antibodies with corresponding concentrations or a blank control were added to the cells in each well, and incubated at 4°C for 0.5 hour, and 100 µL of biotin-conjugated FGF7 at 0.32 µg/mL was then added and incubated at 4°C for 1 hour. After the cells were washed twice with PBS containing 2% FBS, the Alexa 488 streptavidin was added, and incubated at 4°C in dark for 0.5 hour. The cells were washed twice with PBS containing 2% FBS, and then resuspended with 100 µL PBS containing 2% FBS, and finally the fluorescence signal on the cell surface was detected using a flow cytometer, for which 2×10⁴ cells were pipetted per well upon detecting.

**Table 9: The results of determination of the activity of the antibodies in blocking the binding of cell surface receptor FGFR2IIIb to its ligand FGF7 by FACS**

| Antibody No. | ELISA EC₅₀ (nM) | | Maximum inhibition rate (%) | |
|---|---|---|---|---|
| | KATO III | SNU16 | KATO III | SNU16 |
| FWB 1904 | 9.73 | 3.438 | 94.5 | 98.6 |
| FWB 1905 | 9.95 | 3.097 | 93.2 | 98.3 |
| FWB1912 | 10.2 | 4.171 | 82.9 | 87.2 |
| FWB1914 | 8.75 | 3.452 | 93.2 | 98.3 |
| FWB1915 | 9.95 | 3.824 | 91.3 | 97.2 |
| FWB1916 | 20.2 | 6.932 | 95.1 | 97.1 |
| FWB1919 | 12 | 4.997 | 86.3 | 87.5 |
| FWB1921 | 10.95 | 5.202 | 85.7 | 87.8 |
| FWB1925 | 9.84 | 7.855 | 77.5 | 83.0 |

## Claims

1. An antibody or antigen-binding fragment thereof that specifically binds to FGFR2IIIb, comprising heavy chain CDR1, CDR2 and CDR3 and light chain CDR1, CDR2 and CDR3 which have at least 80%, preferably at least 90% identity respectively with the heavy chain CDR1, CDR2 and CDR3 and the light chain CDR1, CDR2 and CDR3 sequences of an antibody selected from the group of FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB1924 and FWB1925.

2. The antibody or antigen-binding fragment thereof that specifically binds to FGFR2IIIb according to claim 1, comprising heavy chain CDR1, CDR2 and CDR3 and light chain CDR1, CDR2 and CDR3 of an antibody selected from the group of: FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB1924 and FWB1925.

3. The antibody or antigen-binding fragment thereof according to claim 1 or 2, comprising a heavy chain variable region and a light chain variable region, wherein the heavy chain variable region and the light chain variable region have at least 85% identity, preferably at least 90% identity, more preferably 95% identity, and most preferably 98% identity with heavy chain variable region and light chain variable region sequences of an antibody selected from the group of: FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB 1922, FWB 1923, FWB1924 and FWB 1925.

4. The antibody or antigen-binding fragment thereof according to claim 3, comprising heavy chain variable region and light chain variable region sequences of an antibody selected from the group of: FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB1924 and FWB1925.

5. The antibody or antigen-binding fragment according to claim 1 or 2, comprising a heavy chain and a light chain, wherein the heavy chain and the light chain have at least 80%, preferably at least 85%, more preferably 90%, most preferably at least 95%, 98%, and 99% identity respectively with heavy chain and light chain sequences of an antibody selected from the group of: FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB1924 and FWB1925.

6. The antibody or antigen-binding fragment thereof according to claim 5, comprising heavy chain and light chain sequences of an antibody selected from the group of: FWB1904, FWB1905, FWB1906, FWB1907, FWB1908, FWB1910, FWB1911, FWB1912, FWB1913, FWB1914, FWB1915, FWB1916, FWB1918, FWB1919, FWB1920, FWB1921, FWB1922, FWB1923, FWB 1924 and FWB 1925.

7. The antibody according to any of claims 1-6, wherein the antibody is a human antibody, a humanized antibody or a chimeric antibody.

8. The antibody according to any of claims 1-6, wherein the antibody is IgA, IgG, and IgD.

9. The antigen-binding fragment according to any of claims 1-6, wherein the antigen-binding fragment is selected from an Fab fragment, an F(ab') fragment, an Fv fragment, an F(ab')2 fragment, a single chain antibody (scFV), and a diabody.

10. An isolated nucleic acid encoding the antibody or antigen-binding fragment thereof according to any of claims 1-9.

11. An expression vector comprising the isolated nucleic acid according to claim 10.

12. A host cell comprising the isolated nucleic acid according to claim 10 or the expression vector according to claim 11, wherein the host cell is preferably a eukaryotic host cell, and more preferably a mammalian host cell.

13. A pharmaceutical composition for treating an FGFR2-related disease or disorder, comprising the antibody or antigen-binding fragment thereof according to claims 1-9, the isolated nucleic acid according to claim 10, the expression vector according to claim 11, or the host cell according to claim 12, and further comprising a pharmaceutical carrier.

14. A composition comprising a first nucleic acid and a second nucleic acid, wherein the first nucleic acid encodes a heavy chain of the antibody according to any of claims 1-9, and the second nucleic acid encodes a light chain of the antibody according to any of claims 1-9.

15. An antibody-drug conjugate comprising the antibody or antigen-binding fragment thereof according to any of claims 1-9 and a drug.

16. Use of the antibody or antigen-binding fragment thereof according to any of claims 1-9 or the antibody-drug conjugate according to claim 15 in the manufacture of a medicament for treating a cancer caused by FGFR2-pathway-related dysregulation, preferably gastric cancer, and more preferably gastric cancer where the FGFR2IIIb gene amplification leads to overexpression of FGFR2IIIb.
